# EUROPÄISCHE PATENTSCHRIFT

(11) **EP 1 661 523 B1**
(45) Veröffentlichungstag und Bekanntmachung des Hinweises auf die Patenterteilung: **09.04.2008**
(21) Anmeldenummer: 04405743.8
(22) Anmeldetag: 30.11.2004
(51) Int. Cl.: A61B 17/64

(54) **Einsatz für ein Klemmelement, Klemmelement mit einem solchen Einsatz und daraus gebildete Gelenkverbindung**
Insert for clamp element, clamp element with such insert and joint formed therewith
Insert pour élément de serrage, élément de serrage avec un tel insert et articulation formée avec ceux-ci.

(43) Veröffentlichungstag der Anmeldung: 31.05.2006
(73) Patentinhaber: Stryker Trauma SA, 2545 Selzach (CH)
(72) Erfinder: Thomke, Roland, 4512 Bellach (CH); Fankhauser, Damian, 3007 Bern (CH)
(74) Vertreter: Liebetanz, Michael

(56) Entgegenhaltungen:
- EP-A- 0 700 664
- WO-A-96/05777
- GB-A- 2 029 490
- US-A- 2 803 349
- US-A- 4 365 624
- US-A- 4 696 293
- US-A- 4 821 382
- US-A- 5 769 556
- 'FAHRRADKINDERSITZ MONTAGE- UND GEBRAUCHSANLEITUNG' GEBRAUCHSANLEITUNG FUER ROEMER JOCKEY CLASSIC 01 April 2003, Seiten 1 - 9, XP002368896

## Beschreibung

### Technisches Gebiet der Erfindung

Die Erfindung betrifft einen Einsatz für ein Klemmelement zum Klemmen eines stabförmigen Elementes einer Gelenkverbindung, insbesondere für ein Klemmelement einer Gelenkverbindung zur Stabilisierung von Knochenbrüchen. Weiterhin betrifft die Erfindung eine Gelenkverbindung mit zwei Klemmelementen und mit einem Verriegelungselement.

### Stand der Technik

Die EP 1 184 000 beschreibt ein einstückiges Klemmelement, das über zwei gegenüberliegende und einen seitlich offenen Hohlraum zur Aufnahme eines stabförmigen Elementes bildende Klemmbacken und ein Scharniermittel verfügt, welches gegenüber dem Hohlraum angeordnet ist, die Klemmbacken miteinander verbindet und dadurch diese aufeinander zu bewegbar sind, wobei jede Klemmbacke jeweils eine Bohrung aufweist, die miteinander fluchtend ausgerichtet sind.

Dieses Klemmelement hat den Vorteil, dass mit zwei nebeneinander angeordneten identischen Klemmelementen eine Gelenkverbindung hergestellt werden kann, wobei dabei durch die genannten Bohrungen eine Verbindungsschraube eingesetzt wird, die in eine innengewindete Mutter eingeschraubt wird, um die Klemmbacken zu schliessen.

Es ist ein Nachteil der bekannten Vorrichtung, dass die stabförmigen Elemente nur von ihren Enden her in Längsrichtung in die aufnehmenden Hohlräume eingeführt werden können.

Aus der US 6,277,069 ist ein anderes Klemmelement bekannt, welches seitlich offen ist. Dies gestattet das seitliche Einlegen eines ersten stabförmigen Elementes. Ein zweites stabförmiges Element ist in eine mit einem Spannhebel verbundene geschlossene Hülse einführbar.

Aus der US 6,616,664 und der EP 0 700 664 ist jeweils eine Gelenkverbindung bekannt, die aus vier einzelnen Klemmbackenelementen und einer zentralen Schraube besteht. Bei dieser Gelenkverbindung ist es möglich, einen oder zwei stabförmige Elemente in die entsprechenden Hohlräume seitlich einzuführen. Bei der EP 0 700 664 ist zwischen den beiden mittleren Klemmbackenelementen eine Feder angeordnet, gegen deren Federkraft es möglich ist, die stabförmigen Elemente einzuklipsen und so vor einer Blockierung der Gelenkverbindung diese an den stabförmigen Elementen festzuhalten. Bei der US 6,616,664 sind schmale, seitlich angeordnete Hebelarme vorgesehen, um seitlich eingelegte stabförmige Elemente vor einer Blockierung der Gelenkverbindung festzuhalten.

Ein Nachteil dieser Vorrichtungen liegt darin, dass die Klemmelemente einer solchen Gelenkverbindung für den Durchmesser des einzusetzenden Stabes oder Stiftes ausgelegt sein müssen. Damit müssen für den Chirurg eine Reihe von unterschiedlichen Klemmelementen zur Verfügung gehalten werden, um den Gegebenheiten einer Operation Rechnung zu tragen.

Weitere Klemmelemente bzw. Einsätze für Klemmelement sind bekannt aus US-A-4 821 382, US-A-2 803 349 bzw. zur Befestigung eines Kinderfahrradsitzes (Römer Jockey Classic, Montage- und Gebrauchsanleitung).

Ausgehend von diesem Stand der Technik ist es eine Aufgabe der vorliegenden Erfindung, einen Einsatz für ein Klemmelement anzugeben, welches das seitliche Einlegen von verschieden dicken stabförmigen Elementen ermöglicht.

Ferner ist es eine Aufgabe der vorliegenden Erfindung, ein einfaches für den Einsatz von solchen Einsätzen konzipiertes Klemmelement anzugeben, welches das seitliche Einlegen von verschieden dicken stabförmigen Elementen ermöglicht, insbesondere mindestens den Einsatz von zwei unterschiedlichen Stäben in zwei Klemmelementen einer Gelenkverbindung.

Ein weiteres Ziel der Erfindung ist es, ein kostengünstiges Einwegklemmelement zu schaffen, insbesondere aus Kunststoff im Spritzguss, welches nachträglich für verschiedene unterschiedliche stabförmige Elemente adaptierbar ist.

Ausgehend vom bekannten Stand der Technik liegt der Erfindung weiterhin die Aufgabe zugrunde, eine verbesserte Gelenkverbindung anzugeben.

### Zusammenfassung der Erfindung

Diese Aufgabe wird erfindungsgemäss für einen Einsatz der eingangs genannten Art mit den kennzeichnenden Merkmalen des Anspruchs 1 gelöst. Ein Kit solcher Einsätze ist in Anspruch 5 gekennzeichnet. Eine erfindungsgemässe Gelenkverbindung ist durch die Merkmale des Anspruchs 6 gekennzeichnet.

Dadurch, dass Klemmelemente nachträglich mit Einsätzen ausgestattet werden können, kann die Grösse der Klemmbacken der zwei Klemmelemente aneinander angepasst werden, um in einfacher Weise für unterschiedliche stabförmige Elemente eines Gelenkelement ausgelegt zu sein.

Andererseits können Klemmelemente bereits mit eingesetzten Einsätzen, in einem oder mehreren der Backen, vorgesehen sein und geliefert werden, die dann bei der tatsächlichen Verwendung in einfacher Weise entfernt werden können.

Wenn die Einsätze aus einem isolierenden Material ausgebildet sind, ist es möglich, die Bildung eines leitenden Kreise über einen externen Fixateur zu vermeiden und somit zur MRI-Sicherheit eines solchen Fixateurs beizutragen.

Ein wesentlicher Vorteil der Klemmelemente nach der Erfindung liegt in der Möglichkeit, durch eine geschickte Materialpaarung einen guten Halt der stabförmigen zu erreichen. Insbesondere kann der Einsatz aus einem weicheren, plastischeren Material als das Klemmelement oder der Stab bestehen, so dass eine erhöhte und verbesserte Haftreibung zwischen Einsatz und Stab und Einsatz und Backe des Klemmelementes auftritt, als es im direkten Kontakt möglich wäre.

Weitere vorteilhafte Ausführungsbeispiele sind in den Unteransprüchen niedergelegt.

### Kurze Figurenbeschreibung

Die Erfindung wird nun unter Bezugnahme auf die Zeichnungen an Hand von Ausführungsbeispielen beispielhaft näher beschrieben.
Es zeigen:
- Fig. 1: eine perspektivische Ansicht eines Gelenkelementes mit zwei Klemmelementen mit einem Einsatz, welcher nicht Teil der beanspruchten Erfindung ist.
- Fig. 2: eine perspektivische Ansicht des Einsatzes nach dem ersten Ausführungsbeispiel für ein Klemmelement nach Fig. 1,
- Fig. 3: eine Seitenansicht des Einsatzes nach Fig. 2,
- Fig. 4: eine perspektivische Darstellung eines Gelenkelementes nach Fig. 1 im blockierten Zustand mit zwei festgeklemmten stabförmigen Elementen und einem Einsatz nach Fig. 2,
- Fig. 5: eine perspektivische Ansicht eines Einsatzes gemäß der Erfindung für ein Klemmelement nach Fig. 1,
- Fig. 6: eine Seitenansicht des Einsatzes nach Fig. 5,
- Fig. 7: eine Seitenansicht eines Gelenkelementes nach Fig. 1 mit einem Einsatz nach Fig. 5,
- Fig. 8: eine perspektivische Ansicht eines Einsatzes nach einem zweiten Ausführungsbeispiel für ein Klemmelement nach Fig. 1,
- Fig. 9: eine Seitenansicht des Einsatzes nach Fig. 8,
- Fig. 10: einen Ausschnitt aus einem externen Fixateur mit mehreren Gelenkelementen und verschiedenen von diesen gehaltenen Stäben unterschiedlichster Konfiguration.

### Ausführliche Beschreibung der bevorzugten Ausführungsbeispiele

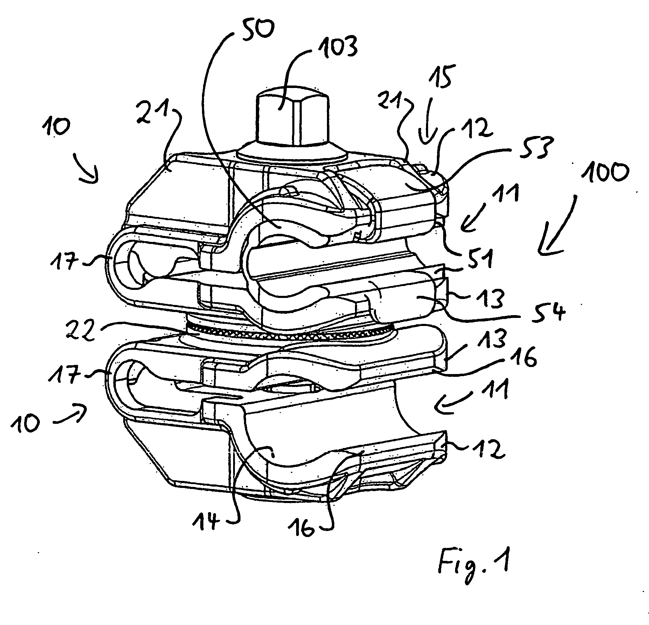

Die Fig. 1 zeigt eine perspektivische Ansicht eines Gelenkelementes 100 mit zwei Klemmelementen 10 mit einem Einsatz 50 Dieses Ausführungsbeispiel ist nicht Teil der Erfindung und lediglich zur Erläuterung genannt. Jedes Klemmelement 10 verfügt über zwei gegenüberliegende und einen Hohlraum 11 zur Aufnahme eines stabförmigen Elementes aufweisen-de Klemmbacken 12 und 13. Die Klemmbacken 12 und 13 verfügen an ihren freien Enden 15 jeweils über eine quer verlaufende Nut 14, die zusammen den Hohlraum 11 aufspannen. An den freien Enden 15 sind die Aussenkanten 16 der aufeinander zugewandten Seite der Klemmbacken 12 und 13 angeschrägt, um das Einschieben eines stabförmigen Elementes von der Seite her zu vereinfachen. Gegenüber dem Hohlraum 11 und den freien Enden 15 ist ein Scharniermittel 17 vorgesehen, welches die Klemmbacken 12 und 13 einstückig miteinander verbindet. Eine Schraube 103,die durch die Klemmelemente 10 hindurchtritt, schliesst das Gelenkelement 100 und verspannt in dieses einlegbare Stäbe. Dabei handelt es sich um ein Verriegelungselement, welches auch über Hebel und andere Elemente realisierbar ist.

Die Klemmelemente 10 weisen im Mittelbereich einen vollen Materialquerschnitt auf, die zwei seitliche Querrippen 21 ausbilden, die insbesondere in dem oberen Bereich der Klemmbacke 12 stark ausgeprägt sind. Der Bereich zwischen den Querrippen 21 ist zur Vorderkante 16 hin bis auf eine in der Draufsicht runde Schraubenaufnähme ausgenommen. Die Schraubenaufnahme verfügt beispielsweise über eine konische Schulterfläche oder über eine Stufenschulter, und geht zur Aufnahme der Schraube 103 in eine durchgehende Bohrung in der oberen Klemmbacke 12 über. Die Rippen können auch vollkommen an der Aussenseite des Klemmelementes verlaufen.

In der unteren Klemmbacke 13 münden die besagten Querrippen 21 in einem Ringflansch 22, der beispielsweise über einen flachen vertieften ringförmigen Absatz verfügen kann, an den sich eine gewichts-, materialsparende und für die Spritzgussherstellung vorteilhafte Ausnehmung anschliessen kann, in deren Mitte eine Bohrung vorgesehen ist. Diese durchgehende Bohrung ist fluchtend mit der oben genannten durchgehenden Bohrung in der oberen Klemmbacke 12 ausgerichtet. Sie verläuft beim Klemmelement 10 senkrecht zur Achse des Hohlraums 11 und parallel zur Rückseite des Scharniermittels 17. Sie könnte aber auch schräg verlaufen.

In der Darstellung der Fig. 1 wird das Gelenkelement 100 aus zwei Klemmelementen 10 gebildet. Das untere Klemmelement 10 ist für einen Stab mit einem Durchmesser von beispielsweise 12 Millimetern vorgesehen. Dann hat die Öffnung an den freien Enden im Ruhezustand einen Durchmesser von beispielsweise 8 Millimetern. Wenn nun das obere Klemmelement 10 für einen Stab mit 4 bis 6 Millimetern Durchmesser vorgesehen sein soll, dann sollte die Öffnung an den freien Enden 15 im Ruhezustand einen Durchmesser von beispielsweise 2 Millimetern aufweisen.

In den Hohlraum 11 ist ein Einsatz 50 eingesetzt, der aus zwei Kurvenflächen 51 in Gestalt von hohlen Viertelkreiszylindern besteht, die über einen Steg 52 miteinander verbunden sind. Diese Abschnitte kann man auch als Mantelelemente 51 bezeichnen. Der Steg 52 ist in radialer Richtung insbesondere dünner als die Kurvenflächen 51. Die Kurvenflächen 51 stossen mit ihren Aussenflächen 56 in Gestalt von äusseren Zylindermänteln an die Innenflächen der Nuten 14 an. Der in der Fig. 1 verwendete Einsatz 50 umfasst zwei Haltezungen 53 und 54, die sich von dem Innenraum 58 des Einsatzes 50 weg erstrecken und die sich insbesondere beim Einsatz mit einem Klemmelement 10 zwischen den besagten Rippen 21 erstrecken. Damit ist der Einsatz 50 gegen axiale Verschiebungen in Richtung des einzusetzenden Stabes gesichert. Durch die beiden Zungen 53 und 54 wird auch ein Verdrehen des Einsatzes sicher vermieden. Die abgerundeten Aussenflächen 55 der Zungen 53, 54 am Übergang in die Kurvenflächen 51 des Einsatzes 50 bilden nun die Einsetzkanten 16 für einen Stab. Die Haltezungen oder Laschen 53 kann man generell auch als Halteabschnitte bezeichnen.

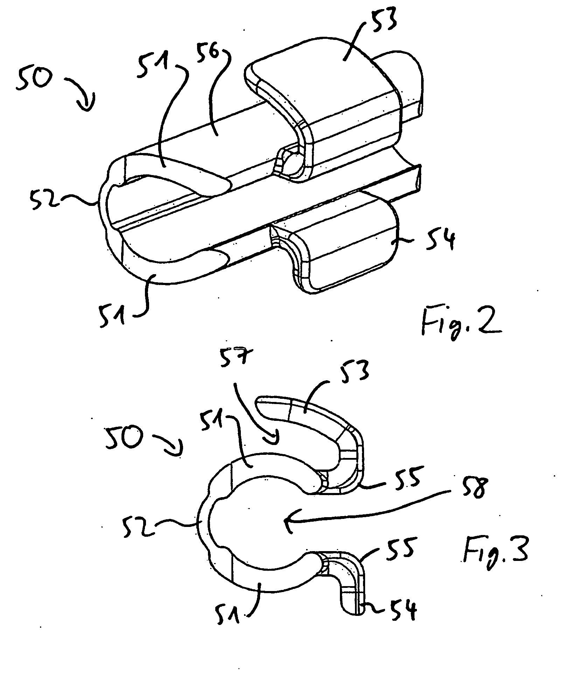

Die Fig. 2 zeigt eine perspektivische Ansicht des Einsatzes 50 für ein Klemmelement 10 nach Fig. 1. Die Fig. 3 zeigt eine Seitenansicht des besagten Einsatzes 50 nach Fig. 2. Der Einsatz 50 verfügt über eine in Richtung des Verbindungssteges 52 ausgerichtete obere Lasche 53, wobei zwischen der Lasche 53 und der Aussenfläche 56 ein Klemmraum 57 vorhanden ist, in den sich ein Teil der oberen Klemmbacke 13 einfügt. Die Lasche 53 kann in der Ruhestellung so ausgebildet sein, nämlich in Richtung Aussenfläche flacher ausgerichtet, dass der Einsatz 50 nach seinem Einsetzen in das Klemmelement 10 von der Lasche 53 unter Vorspannung gehalten wird. Die Länge der oberen Lasche 53 ist hier fast so lang wie die Breite des Viertelzylinders 51. Die untere Lasche 54 ist dagegen wesentlich kürzer und steht zur Achse des Hohlraums 11 senkrecht ausgerichtet. Die Materialdicke der Laschen 53, 54 entspricht denen der Viertelzylinder 51 (hier die Elemente 51 nach Fig. 2 und 3). Die Breite der Laschen 53 und 54 ist vorteilhafterweise zwischen einem Drittel und zwei Drittel der Breite der Viertelzylinder 51. Die Breite der Viertelzylinder 51 ist vorzugsweise in der longitudinalen Dimension der Nut 11 der Backen 12 beziehungsweise 13.

Bei anderen, hier nicht dargestellten Beispielen sind auch andere Laschenformen möglich. Insbesondere kann die obere 53 und/oder die untere 54 Lasche dünner ausgebildet sein und insbesondere komplementär zu einem in der oberen oder unteren Klemmbacke 12 oder 13 vorgesehenen Schlitz. Solch ein Schlitz verläuft dann parallel zur Hauptachse eines einzulegenden Stabes 101, 102. Damit kann der Einsatz ohne überstehende Elemente in Klemmelemente 10 eingedrückt werden und so immer unter Spannung gehalten werden. Der Einsatz 50 ist beispielsweise ein einstückiges Kunststoffelement. Es kann auch ein koextrudiertes Kunststoffelement mit zwei unterschiedlichen Materialarten sein oder ein metallisches Element (als oder in der Lasche) enthalten.

Der Verbindungssteg 52 weist hier den selben Krümmungsradius wie die Flächen 51 auf. Er ist lediglich dünner ausgestaltet und knickt bei Belastung nach vorne in den Hohlraum 11 oder nach hinten aus dem Hohlraum 11 weg.

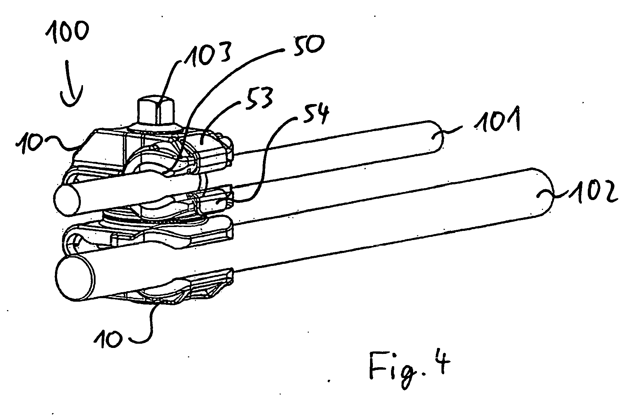

Die Fig. 4 zeigt eine perspektivische Darstellung eines Gelenkelementes 100 nach Fig. 1 im blockierten Zustand mit zwei festgeklemmten stabförmigen Elementen 101 und 102 und einem Einsatz 50 nach Fig. 2.

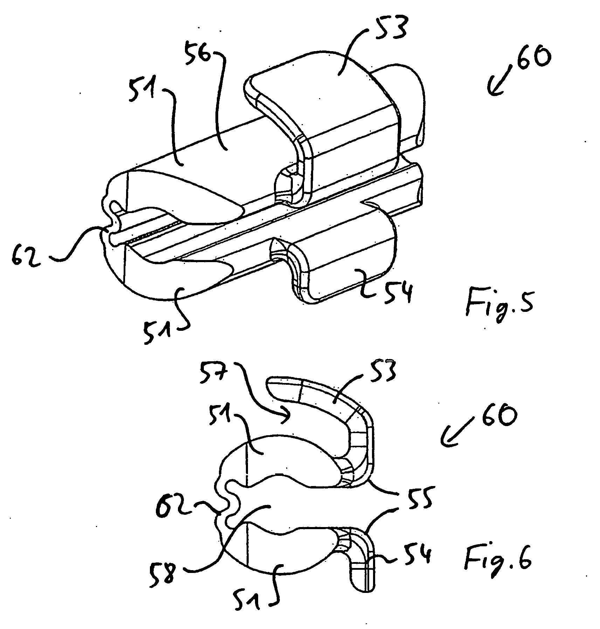

Die Fig. 5 zeigt nun eine perspektivische Ansicht, schräg von oben, eines Einsatzes 60 gemäss der Erfindung Fig. 6 zeigt eine Seitenansicht des Einsatzes 60 nach Fig. 5. Gleiche Merkmale sind jeweils mit gleichen Bezugszeichen versehen. Die zwei wesentlichen Unterschiede zwischen den beiden Einsätzen 50 und 60 nach Fig. 2 und Fig. 5 liegen darin, dass der Einsatz 50 nach Fig. 2 zur Aufnahme eines im Durchmesser grösseren stabförmigen Elementes vorgesehen ist und die Kurvenflächen 51 des Einsatzes 60 nach Fig. 5 eine grössere Materialdicke aufweisen. Nach dem Einsetzen des Einsatzes 60 ist die Mittelachse des Hohlraums 11 aber immer noch in der Höhe des Schlitzes angeordnet. Dies gilt beispielsweise bei einem Einsatz 80, siehe Fig. 14, nicht.

Ferner weist der erfindrische Einsatz 60 nach Fig. 5 einen gewellten Steg 62 auf. Dieser Steg beginnt an den dickeren Viertelzylindern 51 in einer Verlängerung, das heisst in tangentialer Ausrichtung, um dann eine nach innen auf den Hohlraum 58 gebogene Nase zu bilden. Damit besteht ein längerer Federweg für den Steg 62. Die beiden Laschen 53 und 54 sind wie beim ersten Beispiel ausgebildet. Der Steg 82 könnte auch kürzer als die longitudinale Länge des Einsatzes 50 ausgebildet oder unterbrochen sein.

In einem in den Zeichnungen nicht dargestellten Beispiel kann ein Einsatz nach Fig. 2 auch aus zwei getrennten Teilen bestehen, das heisst, es gibt keinen Steg 52. Es sind dann zwei Einsätze verwendbar, von denen beispielsweise einer aus Fläche 51 und Lasche 53 besteht und ein anderer aus Fläche 51 und Lasche 54. Es sind aber auch zwei identische Einsätze möglich. Wesentlich ist dann, dass diese zwei Einsätze unabhängig voneinander oben und unten eingeklickt werden.

Der erfinderische der Fig. 5 ist bei der Verwendung eines kleineren stabförmigen Elementes ausgelegt. Statt dieses einen Einsatzes 60 ist es gemäss einem (in den Zeichnungen nicht dargestellten) Ausführungsbeispiel auch möglich zwei oder mehrere dünne konzentrische Schalen-Einsätze zu verwenden. Darunter wird verstanden, dass zur Erreichung des Effekts eine kleinen Öffnung 58 in einen Einsatz 50 nach Fig. 2, der in der Fig. 1 bereits eingesetzt ist, ein weiterer SchalenEinsatz eingesetzt wird, bei dem es sich um einen konzentrisch zu dem ersten Einsatz 50 ausgerichteten Einsatz handelt. Der innere Schaleneinsatz verfügt dann über eine Fläche 51, die sich mit ihrer Oberseite 56 an die Innenseite des Einsatzes 50 anlehnt, und sie verfügt vorzugsweise über eine Lasche, die sich von aussen auf die erste Lasche 53 legt. Es kann dann noch einen dritten Schaleneinsatz geben, um den Durchmesser der Öffnung 58 um einen weiteren Schritt zu verkleinern. Es wird dabei ein Kit gebildet, dessen verschiedene Einsätze eine konzentrisch ineinander einsetzbare Abfolge von Mantelelementen 51 bilden.

Die hier beschriebenen Ausführungsbeispiele sind in Bezug auf den einzulegenden Stab alle konzentrisch ausgestaltet. Es wird nun hier schon - darauf hingewiesen, dass auch eine entsprechende exzentrische Ausführung des Einsatzes 50 oder 60 möglich ist. Dies bedeutet, dass die Viertelkreiszylinder 51 dann diesen Namen nur noch für Ihre Aussenfläche 56 verdienen, da diese ja weiterhin an eine für ein stabförmiges Element ausgelegte Fläche stossen. Auf der Innenseite kann dann aber eine andere asymmetrische Form vorgesehen sein, im Querschnitt beispielsweise eine Dreiecksnut. Diese gestattet dann das Einsetzen von Stäben verschiedener Grösse, die alle jeweils in Bezug auf eine Ebene gleichartig gelagert werden.

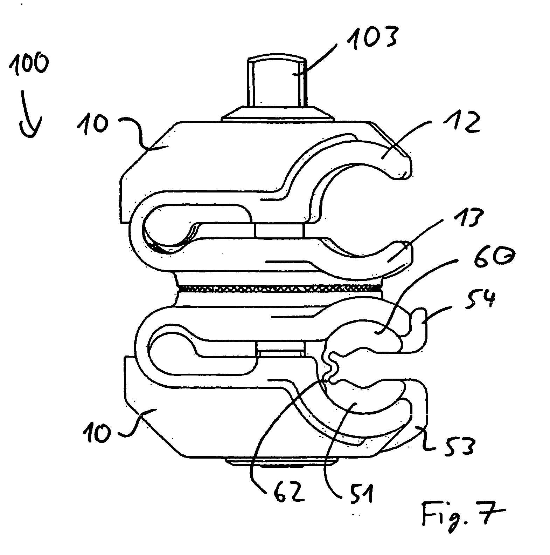

Die Fig. 7 zeigt eine Seitenansicht eines Gelenkelementes 100, wie in Fig. 4 dargestellt, mit einem Einsatz 60 nach Fig. 5 in dem unteren Klemmelement 10.

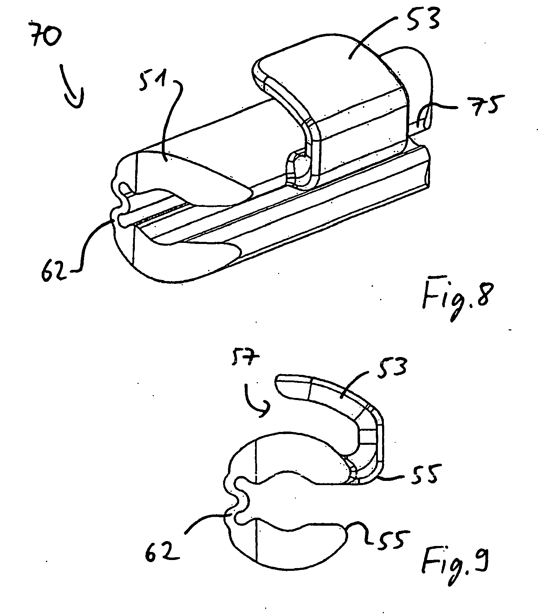

Die Fig. 8 zeigt eine perspektivische Ansicht, schräg von oben, eines Einsatzes 70 gemäss einem zweiten Ausführungsbeispiel. Fig. 9 zeigt eine Seitenansicht des erfindersichen Einsatzes 60 nach Fig. 5. Der einzige Unterschied zwischen den beiden Einsätzen 60 und 70 nach Fig. 5 und Fig. 8 liegt darin, dass der Einsatz 70 nach Fig. 8 nur eine Lasche 53 aufweist. Die Lasche 53 ist für den Eingriff in den Bereich zwischen den Rippen 21 der äusseren Klemmbacke 12 vorgesehen. Somit besteht für die innere Klemmbacke 13 keine Lasche; damit erstreckt sich die äussere Einpressfläche 55 über die gesamte Länge des Einsatzes 70, während an der gegenüberliegenden Seite die Einpressfläche 55 für einen Stab an der Lasche 53 vorsteht und wie bei den vorgängig dargestellten Ausführungsbeispielen links und rechts eine rückstehende Kante 75 besteht.

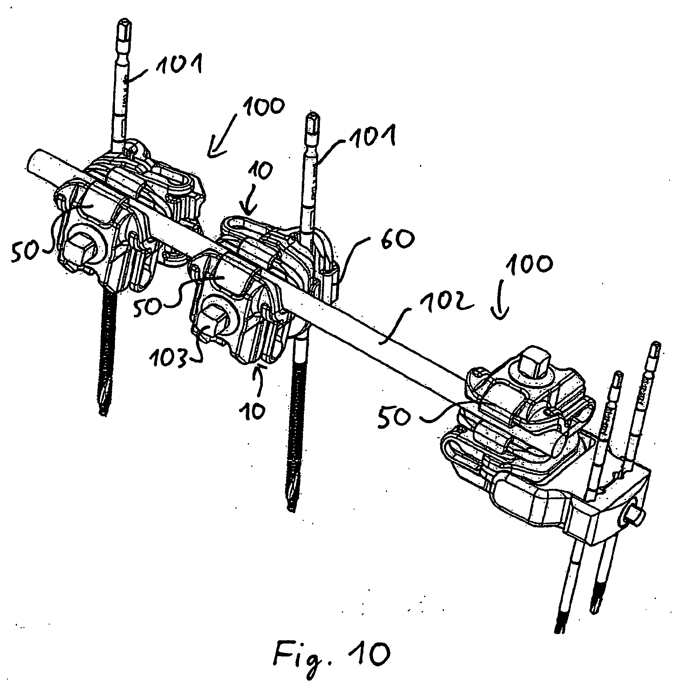

Schliesslich zeigt Fig. 10 einen Ausschnitt aus einem externen Fixateur mit mehreren Gelenkelementen 100 und verschiedenen von diesen gehaltenen Stäben 101, 102 mit verschiedenen Einsätzen 50 und 60 in unterschiedlichster Konfiguration. Die Gelenkelemente 100 sind mit jeweils zwei Klemmelementen 10 aufgebaut, mit denen eine Klemmung eines dicken 10 bis 12 Millimeter Stabes möglich ist. Der hier verwendete mitteldicke Stab 102 hat einen Durchmesser zwischen 6 und 8 Millimeter und erfordert einen Einsatz 50 zur Reduktion und Anpassung des Durchmessers des zu klemmenden Stabes um beispielsweise 2 Millimeter. In das andere Klemmelement 10 wird ein Einsatz 60 zur Reduktion und Anpassung des Durchmessers des zu klemmenden Stabes 101 um beispielsweise 4 Millimeter verwendet, da der hier verwendete dünnere Stab 101 einen Durchmesser zwischen 6 und 8 Millimeter aufweist. Es ist somit klar, dass bei der Verwendung von des Klemmelementes 10 mit keinem, mit einem Einsatz 50 oder mit einem Einsatz 60 drei mögliche Stabdicken verwendbar sind.

Die Materialien der Klemmelemente 10, 20 und der Einsätze 50, 60, 70, können jeweils aus Kunststoff, faserverstärktem Kunststoff, Metall und insbesondere Titan oder einem Stahl gewählt sein. Es ist auch möglich, wie oben erwähnt, Mischungen, insbesondere koextrudierte Elemente zu verwenden. Interessanterweise sind die Einsätze 50, 60, 70, 80, 90 aus einem weicheren Kunststoff, der ein longitudinales Verrutschen oder Verdrehen der Stäbe 101, 102 in dem Einsatz wirksam vermeidet. Dies macht auch die Verwendung von dünneren Einsätzen sinnvoll, die nicht zur Dickenreduktion sondern im wesentlichen oder ausschliesslich zur Haftungsverbesserung eingesetzt werden. Dann sind die besagten Viertelzylinder 51 sehr dünn und nicht dicker als der besagte Steg 52, 62.

Die Laschen 53, 54 sind nicht nur Führungs- und Festlegelemente sondern auch Bedienelemente. Die Laschen 53 und 54 sollen vom Benutzer ergriffen werden können, um die Einsätze 50, 60 etc. zu ergreifen und aus einem Klemmelement 10 herauszunehmen oder einzusetzen.

Es wird hervorgehoben, dass die Bezeichnung Ausführungsbeispiel in der vorgenannten Beschreibung nicht bedeutet, dass nur die zu dem jeweiligen Einsatz, Klemmelement oder Gelenkelement beschriebenen Elemente Gegenstand der Erfindung sind. Es sind dies insbesondere auch Kombinationen aus Merkmalen möglich, die in Gegenständen verschiedener Fig. beschrieben sind. So können Schlitze in den Stegen 52, 62 vorgesehen sein; die Laschen 53, 54 können die gesamte Breite der Einsätze aufweisen und auch geschlitzt sein, um sich um die Rippen 21 herumzulegen; seitlichen Rippen können die Einsätze 50, 60, 70 seitlich begrenzen. Der Schutzbereich ist daher nicht auf die in den Zeichnungen dargestellten Ausführungsbeispiele begrenzt, sondern soll ausschliesslich den beigefügten Ansprüchen entnommen werden können.

### Bezugszeichen

- 10: Klemmelement
- 11: Hohlraum
- 12: Klemmbacke
- 13: Klemmbacke
- 14: quer verlaufende Nut
- 15: freies Ende
- 16: Aussenkante
- 17: Scharniermittel
- 21: Querrippen
- 22: Ringflansch
- 50: Einsatz (erstes Ausführungsbeispiel)
- 51: Viertelkreiszylindern
- 52: Steg
- 53: Haltezunge
- 54: Haltezunge
- 55: Aussenfläche
- 56: Aussenmantelfläche
- 57: Klemmraum
- 58: Innenraum
- 60: Einsatz (erstes Ausführungsbeispiel)
- 62: gewellter Steg
- 70: Einsatz (zweites Ausführungsbeispiel)
- 75: seitliche Aussenfläche
- 100: Gelenkelement
- 101: dünnes stabförmige Element
- 102: dickes stabförmige Element
- 103: Schraube

## Patentansprüche

1. Einsatz (60, 70) für ein Klemmelement (10, 20) mit zwei Backen (12, 13) zum Klemmen eines stabförmigen Elementes (101, 102), mit zwei Mantelelementen (51), welche in jeweils eine Backe (12, 13) des Klemmelementes (10, 20) einsetzbar sind, um den für das stabförmige Element (101, 102) verfügbaren freien Raum zu verändern, insbesondere zu verkleinern, und mit einem Halteabschnitt (53, 54), welcher den Einsatz oder einen Teil des Einsatzes (60, 70) in oder an mindestens einer der Backen (12, 13) des Klemmelementes fixiert, wobei die Mantelelemente (51) über einen verbindungssteg (62) miteinander verbunden sind, **dadurch gekennzeichnet, dass** der Verbindungssteg ein Mäandersteg (62) ist.

2. Einsatz nach Anspruch 1, **dadurch gekennzeichnet, dass** der Mäandersteg (62) ein im Querschnitt gewellter Steg ist, der eine innen auf den Raum (58) zwischen den Mantelelementen (51) gebogene Nase aufweist.

3. Einsatz nach einem der Ansprüche 1 oder 2, **dadurch gekennzeichnet, dass** der oder die Halteabschnitte Laschen (53, 54) sind, und dass Teile der Backen (12, 13) der Klemmelemente (10, 20) zwischen dem zugeordneten Mantelelement (51) und der Lasche (53, 54) im Formschluss oder unter Vorspannung umgeben sind.

4. Einsatz nach einem der Ansprüche 1 bis 3, **dadurch gekennzeichnet, dass** eines der Mantelelemente (51) an seiner Innenseite keine bezüglich Seiner Aussenmantelfläche (56) konzentrische Form aufweist, insbesondere an seiner Innenseite über eine im Querschnitt Dreicksform aufweist.

5. Kit von Einsätzen nach einem der Ansprüche 1 bis 4, **dadurch gekennzeichnet, dass** die Einsätze eine konzentrisch ineinander einsetzbare Abfolge von Mantelelementen (51) bilden.

6. Gelenkelement (100, 110) mit zwei Klemmelementen (10, 20), wobei die Klemmelemente mit ihren ersten Klemmbacken (13) aufeinander ausgerichtet übereinander angeordnet sind, und mit mindestens einem Einsatz (60, 70) nach einem der Ansprüche 1 bis 4.

## Claims

1. Insert (60, 70) for a clamping element (10, 20) with two jaws (12, 13) for the clamping of a rod-shaped element (101, 102), comprising two jacket elements (51), each provided to be inserted in a jaw (12, 13) of the clamping element (10, 20), in order to modify, particularly to reduce, the space available for the rod-shaped element (101, 102), and having a retaining section (53, 54), which fixes the insert or a portion of the insert (60, 70) in or on at least one of the jaws (12, 13) of the clamping element, wherein the jacket elements (51) are connected to each other by means of a connecting web (62), **characterized in that** the connecting web is a meander web (62).

2. Insert as claimed in Claim 1, **characterized by** the fact that the meander web (62) is a web being corrugated in cross-section comprising a nose directed and folded towards the room (58) between the jacket elements (51).

3. Insert as claimed in Claim 1 or 2, **characterized by** the fact that the retaining section(s) are clips (53, 54), and that portions of the jaws (12, 13) of the clamping elements (10, 20) between the assigned jacket element (51) and the clip (53, 54) are enclosed in a positive fit or under tension.

4. Insert as claimed in one of Claims 1 to 3, **characterized by** the fact that one of the jacket elements (51) does not have a shape on its inner side that is concentric to its outer jacket surface (56), in particular on its inner side has a shape that is triangular in its cross-section.

5. Kit of inserts as claimed in one of Claims 1 to 4, **characterized by** the fact that the inserts form a sequence of jacket elements (51) that may be inserted concentrically into one another.

6. Articulated element (100, 110) with two clamping elements, whereby the clamping elements (10, 20) are oriented with their first clamping jaws (13) toward each other and arranged one on top of the other, and comprising at least one insert (60, 70) as claimed in Claims 1 to 4.

## Revendications

1. Insert (60, 70) pour un élément de serrage (10, 20) avec deux mâchoires (12, 13) pour serrer un élément en forme de barre (101, 102) comprenant deux éléments de gaine (51), chacun prévu pour être mis en place dans une mâchoire (12, 13) de l'élément de serrage (10, 20) pour modifier, particulièrement pour réduire l'espace libre disponible pour l'élément en forme de barre (101, 102) et possédant une section de support (53, 54) qui fixe l'insert ou une portion de l'insert (60, 70) dans ou sur une des mâchoires (12, 13) de l'élément de serrage, où les éléments de gaine (51) sont reliés ensemble par une âme (62) connecteur, **caractérisé en ce que** l'âme connecteur est une âme en méandre (62).

2. Insert selon la revendication 1, **caractérisé en ce que** l'âme en méandre (62) est, en coupe transversale, une âme ondulée, comprenant un nez, dirigé et plié envers l'espace (58) entre les éléments de gaine (51).

3. Insert selon la revendication 1 ou 2, **caractérisé en ce que** les sections de support sont des pattes (53, 54) et **en ce que** des portions des mâchoires (12, 13) des éléments de serrage (10, 20) entre l'élément de gaine (51) associé et la patte (53, 54) sont dans un maintien positif ou sous tension.

4. Insert selon l'une des revendications 1 à 3, **caractérisé en ce qu'**un des éléments de gaine (51) n'a pas, sur sa face intérieure, une forme concentrique par rapport à la surface de gaine extérieure (56), en particulier que la partie intérieure a une forme qui est, en coupe transversale, triangulaire.

5. Kit d'inserts selon l'une des revendications 1 à 4, **caractérisé en ce que** les inserts forment une séquence d'éléments de gaine (51), éléments qui peut être emboités d'une manière concentrique les un dans les autres.

6. Elément d'articulation (100, 110) avec deux éléments de serrage (10, 20), où les éléments de serrage sont orientés, avec leur premières mâchoires (13), l'un vers l'autre et arrangés un sur l'autre et comprenant au moins un insert (60, 70) selon l'une des revendications 1 à 4.
